# EUROPEAN PATENT APPLICATION

(11) **EP 4 686 474 A1**
(43) Date of publication of application: **04.02.2026**
(21) Application number: 24217181.7
(22) Date of filing: 03.12.2024
(51) Int. Cl.: A61K 31/40, A61K 31/403, A61K 31/4162, A61K 31/4196, A61K 31/4745, A61K 31/496, A61K 31/4985, A61K 31/506, A61K 31/513, A61K 31/519, A61K 31/522, A61P 1/16

(54) **DPP-IV INHIBITORS FOR THE TREATMENT OR PREVENTION OF AUTOIMMUNE LIVER DISEASES**

(30) Priority: 31.07.2024 EP 24306289
(71) Applicant: Delta 4 GmbH, 1080 Vienna (AT); 4P-Pharma, 59000 Lille (FR)
(72) Inventor: MARTIN, Céline, 59000 Lille (FR); SIBRAN, William, 59000 Lille (FR); PERCO, Paul, 1080 Vienna (AT); FILLINGER, Lucas, 1080 Vienna (AT); GEBESHUBER, Christoph, 1080 Vienna (AT)
(74) Representative: Icosa

(57) **Abstract**

The present invention relates to an inhibitor of dipeptidyl peptidase-IV enzyme for use in treating or preventing autoimmune liver diseases and to a composition for use in treating or preventing autoimmune liver diseases comprising at least one inhibitor of dipeptidyl peptidase-IV enzyme.

## Description

### FIELD OF INVENTION

The present invention relates to the field of autoimmune liver diseases, in particular, to compounds for use in treating or preventing autoimmune liver diseases (AILDs). The present invention relates to inhibitors of dipeptidyl peptidase-IV enzyme (DPP-IV inhibitors) for use in treating or preventing autoimmune liver diseases such as autoimmune hepatitis (AIH), primary biliary cholangitis (PBC), primary sclerosing cholangitis (PSC), autoimmune cholangitis (AIC), autoimmune sclerosing cholangitis (ASC) and/or IgG4-related autoimmune cholangitis (IAC).

### BACKGROUND OF INVENTION

Autoimmune liver diseases refer to a group of liver disorders, wherein the immune system attacks the liver, causing inflammation. Examples of autoimmune liver diseases include autoimmune hepatitis (AIH), primary biliary cholangitis (PBC), and primary sclerosing cholangitis (PSC).

Cholestatic liver diseases are a group of diseases characterized by jaundice and cholestasis. Cholestasis is the slowing or interruption of bile flow from the liver. In a healthy subject the liver produces bile, which is then distributed to the digestive system through a network of bile ducts located either inside the liver (intrahepatic ducts) or outside of it (extrahepatic ducts). The liver, the bile ducts and the organs to which the bile is distributed (gallbladder, small intestine and pancreas) constitute the biliary system.

In case of cholestasis, the bile accumulates in the bile ducts and organs, irritating the tissues and interfering with their function. Cholestasis can be a temporary problem or part of an ongoing and worsening disease. Such chronic cholestatic diseases may occur in infancy, childhood or adulthood.

Primary biliary cholangitis (PBC) and primary sclerosing cholangitis (PSC) are two major types of chronic cholestatic liver disease. Both can lead to cirrhosis and hepatic failure, leaving patients in need of a liver transplantation.

Primary biliary cholangitis (PBC) is a complex, chronic, and slowly progressive autoimmune liver disease. PBC is thought to be triggered by environmental factors in genetically susceptible individuals. As the disease progresses, the bile duct deteriorates leading to an impaired secretion and the accumulation of bile acid toxins inside the liver.

Primary sclerosing cholangitis (PSC) is an idiopathic cholestatic hepatobiliary disease in which the medium and large-sized extra and / or intrahepatic bile ducts narrow, sometimes to the point where they are fully obstructed. This obstruction causes a deterioration of the liver.

No cure for these diseases is currently available, as they can even re-occur after a liver transplant. Thus, there is a need for efficient treatment and/or prevention of chronic cholestatic liver diseases, such as PBC or PSC, in order to stop the progression of the disease before the organs are permanently damaged.

Autoimmune hepatitis (AIH) is a chronic liver disease, characterized by elevation of aminotransferases, presence of anti-nuclear antibody or anti-smooth muscle antibody, elevated immunoglobulin G (IgG), and interface hepatitis/plasma-lymphocytic inflammation. It may progress to liver cirrhosis and liver failure.

Progression of AIH may be slowed or stopped with prednisone in association with immunosuppressive treatment. However, this treatment is associated with a range of serious effects, including diabetes, weakened or broken bones, high blood pressure, cataracts, glaucoma, and weight gain. Thus, there is also a need for efficient treatment and/or prevention of autoimmune hepatitis with lower side effects.

Surprisingly, it has been found that a class of known diabetic medications, the DPP-IV inhibitors, also known as gliptins, are good candidates for treating and preventing autoimmune liver diseases.

### SUMMARY

The present invention relates to an inhibitor of dipeptidyl peptidase-IV enzyme; for use in treating or preventing an autoimmune liver disease, preferably a chronic cholestatic liver disease such as primary biliary cholangitis (PBC) and/or primary sclerosing cholangitis (PSC) in a subject in need thereof, wherein the inhibitor of dipeptidyl peptidase-IV enzyme is a compound selected from the group consisting of formula (I), alogliptin, anagliptin, besigliptin, denagliptin, evogliptin, gemigliptin, gosogliptin, linagliptin, omarigliptin, saxagliptin, teneligliptin, trelagliptin, vildagliptin, and any pharmaceutically acceptable salts, solvates and mixtures thereof, wherein said formula (I) is as follows: 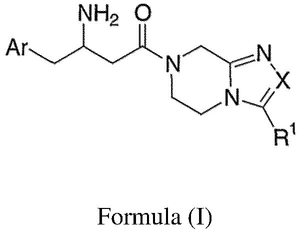 wherein:
Ar is phenyl which is unsubstituted or substituted with 1 to 5 substituents which are similar or different and independently selected from the group consisting of:
   (1) halogen,
   (2) C1-C₆ alkyl, which is linear or branched and is unsubstituted or substituted with 1 to 5 halogens,
   (3) OC1-C₆ alkyl, which is linear or branched and is unsubstituted or substituted with 1 to 5 halogens, and
   (4) CN;
X is selected from the group consisting of: N, and CR²;
R¹ and R² are independently selected from the group consisting of:
   (1) hydrogen,
   (2) CN,
   (3) C₁-C₁₀ alkyl, which is linear or branched and which is unsubstituted or substituted with 1 to 5 halogens or phenyl, which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched,
   (4) phenyl which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched, and
   (5) a 5- or 6-membered heterocycle which may be saturated or unsaturated comprising 1 to 4 heteroatoms independently selected from N, S and O, the heterocycle being unsubstituted or substituted with 1 to 3 substituents independently selected from oxo, OH, halogen, C₁-C₆ alkyl, and OC₁-C₆ alkyl, wherein the C₁-C₆ alkyl and OC₁-C₆ alkyl are linear or branched and optionally substituted with 1 to 5 halogens;
R³ is C₁-C₆ alkyl, which is linear or branched and which is unsubstituted or substituted with 1 to 5 groups independently selected from halogen, CO₂H, and CO₂C₁-C₆ alkyl, wherein the CO₂C₁-C₆ alkyl is linear or branched.

In some embodiments, the inhibitor of dipeptidyl peptidase-IV enzyme is a compound of formula (I) or any pharmaceutically acceptable salts, solvates or mixtures thereof, wherein said formula (I) is as follows: 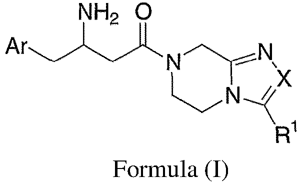 wherein:
Ar is phenyl which is unsubstituted or substituted with 1 to 5 substituents which are similar or different and independently selected from the group consisting of:
   (1) halogen,
   (2) C₁-C₆ alkyl, which is linear or branched and is unsubstituted or substituted with 1 to 5 halogens,
   (3) OC₁-C₆ alkyl, which is linear or branched and is unsubstituted or substituted with 1 to 5 halogens, and
   (4) CN;
X is selected from the group consisting of: N, and CR²;
R¹ and R² are independently selected from the group consisting of:
   (1) hydrogen,
   (2) CN,
   (3) C₁-C₁₀alkyl, which is linear or branched and which is unsubstituted or substituted with 1 to 5 halogens or phenyl, which is unsubstituted or substituted with 1 to 5 substituents independently selected from halogen, CN, OH, R³, OR³, NHSO₂R³, SO₂R³, CO₂H, and CO₂C₁-C₆alkyl, wherein the CO₂C₁-C₆alkyl is linear or branched,
   (4) phenyl which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched, and
   (5) a 5- or 6-membered heterocycle which may be saturated or unsaturated comprising 1 to 4 heteroatoms independently selected from N, S and O, the heterocycle being unsubstituted or substituted with 1 to 3 substituents independently selected from oxo, OH, halogen, C₁-C₆ alkyl, and OC₁-C₆ alkyl, wherein the C₁-C₆ alkyl and OC₁-C₆ alkyl are linear or branched and optionally substituted with 1 to 5 halogens;
R³ is C₁-C₆ alkyl, which is linear or branched and which is unsubstituted or substituted with 1 to 5 groups independently selected from halogen, CO₂H, and CO₂C₁-C₆alkyl, wherein the CO₂C₁-C₆alkyl is linear or branched.

In some embodiments, the inhibitor of dipeptidyl peptidase-IV enzyme is a compound of formula (I), wherein:
- Ar is a phenyl which is unsubstituted or substituted with 1 to 5 substituents which are similar or different and independently selected from the group consisting of fluoro, bromo and CF₃;
- R¹ is selected from the group consisting of: hydrogen and C₁-C₆alkyl which is linear or branched and which is unsubstituted or substituted with phenyl or 1 to 5 fluoro;
- X is selected from the group consisting of: N, and CR²;
- R² is selected from the group consisting of: hydrogen, C₁-C₆ alkyl which is linear or branched and which is unsubstituted or substituted with 1 to 5 fluoro, and phenyl which is unsubstituted or substituted with 1 to 3 substituents independently selected from fluoro, OCH₃, and OCF₃.

In some embodiments, the inhibitor of dipeptidyl peptidase-IV enzyme is a compound of formula (I), wherein :
- Ar is selected from the group consisting of: phenyl, 2-fluorophenyl, 3,4-difluorophenyl, 2,5-difluorophenyl, 2,4,5-trifluorophenyl, 2-fluoro-4-(trifluoromethyl)phenyl, and 4-bromo-2,5-difluorophenyl;
- R¹ is selected from the group consisting of: hydrogen, methyl, ethyl, CF₃, CH₂CF₃, CF₂CF₃, phenyl, and benzyl;
- X is selected from the group consisting of: N, and CR²;
- R² is selected from the group consisting of: hydrogen, methyl, ethyl, CF₃, CH₂CF₃, CF₂CF₃phenyl, (4-methoxy)phenyl, (4-trifluoromethoxy)phenyl, 4-fluorophenyl, and 3,4-difluorophenyl.

In some embodiments, the inhibitor of dipeptidyl peptidase-IV enzyme is a compound of formula (I), wherein:
- Ar is selected from the group consisting of: phenyl, 2-fluorophenyl, 3,4-difluorophenyl, 2,5-difluorophenyl, 2,4,5-trifluorophenyl, 2-fluoro-4-(trifluoromethyl)phenyl, and 4-bromo-2,5-difluorophenyl;
- R¹ is selected from the group consisting of: hydrogen and CF₃;
- X is selected from the group consisting of: N, and CR²;
- R² is selected from the group consisting of: CF₃ and CF₂CF₃.

In some embodiments, the inhibitor of dipeptidyl peptidase-IV enzyme is selected from the group consisting of: alogliptin, anagliptin, besigliptin, denagliptin, evogliptin, gemigliptin, gosogliptin, linagliptin, omarigliptin, saxagliptin, sitagliptin, teneligliptin, trelagliptin, vildagliptin, and any pharmaceutically acceptable salts, solvates and mixtures thereof.

In some embodiments, the inhibitor of dipeptidyl peptidase-IV enzyme is selected from the group consisting of: alogliptin, linagliptin, saxagliptin, sitagliptin, vildagliptin and any pharmaceutically acceptable salts, solvates and mixtures thereof.

In some embodiments, the inhibitor of dipeptidyl peptidase-IV enzyme is sitagliptin or any pharmaceutically acceptable salts or solvates thereof.

In some embodiments, the inhibitor of dipeptidyl peptidase-IV enzyme is to be administered before, concomitantly with or after an additional therapeutic agent.

In some embodiments, the subject is a child, a teenager, or an adult.

The present invention also relates to a pharmaceutical composition for use in treating or preventing an autoimmune liver disease in a subject in need thereof, wherein the pharmaceutical composition comprises at least one inhibitor of dipeptidyl peptidase-IV enzyme, wherein said inhibitor of dipeptidyl peptidase-IV enzyme is selected from the group consisting of: alogliptin, anagliptin, besigliptin, denagliptin, evogliptin, gemigliptin, gosogliptin, linagliptin, omarigliptin, saxagliptin, teneligliptin, trelagliptin, vildagliptin, a compound of formula (I), and any pharmaceutically acceptable salts, solvates and mixtures thereof, wherein said formula (I) is as follows: 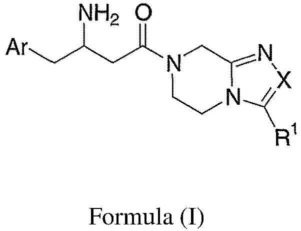 wherein:
Ar is phenyl which is unsubstituted or substituted with 1 to 5 substituents which are similar or different and independently selected from the group consisting of:
   (1) halogen,
   (2) C₁-C₆ alkyl, which is linear or branched and is unsubstituted or substituted with 1 to 5 halogens,
   (3) OC₁-C₆ alkyl, which is linear or branched and is unsubstituted or substituted with 1 to 5 halogens, and
   (4) CN;
X is selected from the group consisting of: N, and CR²;
R¹ and R² are independently selected from the group consisting of:
   (1) hydrogen,
   (2) CN,
   (3) C₁-C₁₀ alkyl, which is linear or branched and which is unsubstituted or substituted with 1 to 5 halogens or phenyl, which is unsubstituted or substituted with 1 to 5 substituents independently selected from halogen, CN, OH, R³, OR³, NHSO²R³, SO₂R³, CO₂H, and CO₂C₁-C₆ alkyl, wherein the CO₂C₁-C₆ alkyl is linear or branched,
   (4) phenyl which is unsubstituted or substituted with 1 to 5 substituents independently selected from halogen, CN, OH, R³, OR³, NHSO₂R³, SO₂R³, CO₂H, and CO₂C₁-C₆alkyl, wherein the CO₂C₁-C₆ alkyl is linear or branched, and
   (5) a 5- or 6-membered heterocycle which may be saturated or unsaturated comprising 1 to 4 heteroatoms independently selected from N, S and O, the heterocycle being unsubstituted or substituted with 1 to 3 substituents independently selected from oxo, OH, halogen, C₁-C₆ alkyl, and OC₁-C₆ alkyl, wherein the C₁-C₆ alkyl and OC₁-C₆ alkyl are linear or branched and optionally substituted with 1 to 5 halogens;
R3 is C₁-C₆ alkyl, which is linear or branched and which is unsubstituted or substituted with 1 to 5 groups independently selected from halogen, CO₂H, and CO₂C₁-C₆ alkyl, wherein the CO₂C₁-C₆ alkyl is linear or branched.

In some embodiments, the pharmaceutical composition further comprises at least one pharmaceutically acceptable excipient selected from the group consisting of: ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat.

In some embodiments, the autoimmune liver disease as described herein is a chronic cholestatic liver disease and/or autoimmune hepatitis.

In some embodiments, the chronic cholestatic liver disease as described herein is primary biliary cholangitis (PBC) and/or primary sclerosing cholangitis (PSC).

### DEFINITIONS

In the present invention, the following terms have the following meanings:

The terms **"a"** and **"an"** refer to one or to more than one (*i.e.,* to at least one) of the grammatical object of the article. By way of example, "an element" means one element or more than one element.

**"About"** preceding a figure encompasses plus or minus 10%, or less, of the value of said figure. It is to be understood that the value to which the term "about" refers is itself also specifically, and preferably, disclosed.

The term **"alkyl"** as used herein by itself or as part of another substituent refers to a hydrocarbyl radical of formula CₙH₂ₙ₊₁ wherein n is a number greater than or equal to 1; which may thus comprise, for example, C₁ to C₁₂ alkyls, such as C₁, C₂, C₃, C₄, C₅, C₆, C₇, C₈, C₉, C₁₀, C₁₁ or C₁₂ alkyls. Generally, alkyl groups of this invention comprise from 1 to 8 carbon atoms, preferably from 1 to 6 carbon atoms, more preferably from 1 to 4 carbon atoms, more preferably from 1 to 3 carbon atoms. Alkyl groups may be linear or branched. Suitable alkyl groups include methyl, ethyl, propyl, butyl, pentyl, hexyl, heptyl and octyl.

**"At least one"** refers to one, two, three or more.

**"Cholestatic"** refers to a disease or syndrome in which there is a stagnation, or at least a marked reduction, in bile secretion and flow (cholestasis).

**"Comprising"** or **"comprise"** is to be construed in an open, inclusive sense, but not limited to. In an embodiment, "comprising" means "consisting essentially of". In an embodiment, "comprising" means "consisting of", which is to be construed as limited to.

**"Dipeptidyl peptidase-4"** or **"DPP-IV"** is a serine protease that is expressed on the surface of epithelial cells in various tissues, including liver, fibroblasts, as well as immune cells like T-cells, B-cells, natural killer cells, dendritic cells, and macrophages. DPP-IV is responsible for the degradation of glucagon-like peptide 1 (GLP-1), a peptide hormone secreted by enteroendocrine L cells that modulates activities of different cells by interacting with the G-protein coupled receptor GLP-1 receptor (GLP1 R). GLP-1 R signaling is associated with glucose homeostasis.

**From X to Y"** refers to the range of values between X and Y, the limits X and Y being included in said range.

The term " **"halogen"** refers in particular to fluorine (F), chlorine (Cl), bromine (Br), or iodine (I) atoms; hence the term **"halo"** also refers to fluoro, chloro, bromo or iodo functional groups.

The term **"heteroatom"** refers to any atom that is not carbon or hydrogen.

The terms **"heterocyclyl"** or **"heterocycle** " as used herein by itself or as part of another group refer to non-aromatic, fully saturated or partially unsaturated cyclic groups (for example, 3 to 7 members monocyclic, 7 to 1 1 member bicyclic, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system, where valence allows. The rings of multi-ring heterocycles may be fused, bridged and/or joined through one or more spiro atoms. An optionally substituted heterocyclic refers to a heterocyclic having optionally one or more substituent(s) (for example 1 to 4 substituent(s), or for example 1, 2, 3 or 4 substituent(s)), selected from those defined above for substituted aryl. Non limiting exemplary heterocyclic groups include aziridinyl, oxiranyl, thiiranyl, piperidinyl, azetidinyl, 2-imidazolinyl, pyrazolidinyl imidazolidinyl, isoxazolinyl, oxazolidinyl, isoxazolidinyl, thiazolidinyl, isothiazolidinyl, piperidinyl, succinimidyl, 3H-indolyl, indolinyl, isoindolinyl, 2H-pyrrolyl, 1-pyrrolinyl, 2-pyrrolinyl, 3-pyrrolinyl, pyrrolidinyl, 4H-quinolizinyl, 2-oxopiperazinyl, piperazinyl, homopiperazinyl, 2-pyrazolinyl, 3-pyrazolinyl, tetrahydro-2H-pyranyl, 2H-pyranyl, 4H-pyranyl, 3,4-dihydro-2H-pyranyl, oxetanyl, thietanyl, 3-dioxolanyl, 1,4-dioxanyl, 2,5-dioximidazolidinyl, 2-oxopiperidinyl, 2-oxopyrrolodinyl, indolinyl, tetrahydropyranyl, tetrahydrofuranyl, tetrahydrothiophenyl, tetrahydroquinolinyl, tetrahydroisoquinolin-1-yl, tetrahydroisoquinolin-2-yl, tetrahydroisoquinolin-3-yl, tetrahydroisoquinolin-4-yl, thiomorpholin-4-yl, thiomorpholin-4-ylsulfoxide, thiomorpholin-4-ylsulfone, 1,3-dioxolanyl, 1,4-oxathianyl, 1,4-dithianyl, 1,3,5-trioxanyl, 1H-pyrrolizinyl, tetrahydro-1,1-dioxothiophenyl, N-formylpiperazinyl, and morpholin-4-yl.

**"Inhibitor of dipeptidyl peptidase-IV enzyme"** refers to a natural or synthetic compound/molecule that has a biological effect to inhibit or significantly reduce the enzymatic activity of said dipeptidyl peptidase-IV enzyme, and/or to inhibit or significantly reduce the expression of a functional dipeptidyl peptidase-IV enzyme.

**"Pharmaceutically acceptable excipient"** or **"pharmaceutically acceptable carrier"** refers to an excipient or carrier that does not produce an adverse, allergic or other untoward reaction when administered to a mammal, preferably a human. It includes any and all solvents, such as, for example, dispersion media, coatings, antibacterial and antifungal agents, isotonic and absorption delaying agents. A pharmaceutically acceptable excipient or carrier refers to a non-toxic solid, semi-solid or liquid filler, diluent, encapsulating material or formulation auxiliary of any type. For human administration, preparations should meet sterility, pyrogenicity, general safety and purity standards as required by the regulatory offices such as the FDA (US Food and Drug Administration) or EMA (European Medicines Agency).

**"Pharmaceutical composition"** refers to the combination of at least one active agent and at least one pharmaceutically acceptable excipient.

**"Primary biliary cholangitis"** or **"Primary biliary cirrhosis"** (PBC) refers to a chronic disease in which the small bile ducts in the liver become inflamed and may eventually be destroyed. Thus, leading to a buildup of bile that causes liver damage. Over time, such liver damage may lead to liver scarring, cirrhosis, and even liver failure.

**"Primary sclerosing cholangitis"** (PSC): refers to a chronic liver disease in which the bile ducts inside and outside the liver becomes inflamed and/or scarred, eventually leading to the bile ducts been narrowed or blocked.

The term "salt" of the compounds of the invention is used herein to describe the acid addition and base salts thereof. Suitable acid addition salts are formed from acids which form non-toxic salts. Non-limiting examples include the acetate, trifluoroacetate, adipate, aspartate, benzoate, besylate, bicarbonate/carbonate, bisulphate/sulphate, borate, tetrafluoroborate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

The term **"solvate"** is used herein to describe a compound in this invention that contains stoichiometric or sub-stoichiometric amounts of one or more pharmaceutically acceptable solvent molecule such as ethanol or water. The term **"hydrate"** refers to when the said solvent is water.

**Subject"** refers to a warm-blooded animal, more preferably a mammal. The term "mammal" refers here to any mammal, including humans. Preferably, the mammal is a primate, more preferably a human. Hence, the term may refer to humans and non-human mammals.

**"Therapeutically effective amount"** means a level or amount of agent that is aimed at, without causing significant negative or adverse side effects to the target, (1) delaying or preventing the onset of a disease, disorder, or condition; (2) slowing down or stopping the progression, aggravation, or deterioration of one or more symptoms of the disease, disorder, or condition; (3) bringing about ameliorations of the symptoms of the disease, disorder, or condition; (4) reducing the severity or incidence of the disease, disorder, or condition; or (5) curing the disease, disorder, or condition. A therapeutically effective amount may be administered prior to the onset of the disease, disorder, or condition, for a prophylactic or preventive action. Alternatively, or additionally, the therapeutically effective amount may be administered after initiation of the disease, disorder, or condition, for a therapeutic action.

**"Treating"** or **"Treatment"** refers to a therapeutic treatment, or a reduction or alleviation and/or slow down of one or more of the symptoms or manifestations of a disease.

**"Preventing"** or **"Prevention"** refers to a prophylactic (or preventative) treatment, or to prevent one or more of the symptoms or manifestations of a disease. The term may thus refer, in particular, to a **"reduction of the likelihood of occurrence"** or a **"reduction of the likelihood of re-occurrence"** of a given condition (e.g. an autoimmune liver disease, a cholestatic liver disease, and/or a condition related thereto), or the one or more symptoms or manifestations of the disease.

**"Treating and preventing"** refers to a therapeutic treatment and a prophylactic (or preventative) treatment.

### DETAILED DESCRIPTION

This invention relates to an inhibitor of dipeptidyl peptidase-IV enzyme (DPP-IV) for use in treating or preventing an autoimmune liver disease in a subject in need thereof, wherein the inhibitor of dipeptidyl peptidase-IV enzyme is a compound selected from the group comprising or consisting of formula (I), alogliptin, anagliptin, besigliptin, denagliptin, evogliptin, gemigliptin, gosogliptin, linagliptin, omarigliptin, saxagliptin, teneligliptin, trelagliptin, vildagliptin and any pharmaceutically acceptable salts, solvates and mixtures thereof, wherein said formula (I) is as follows: 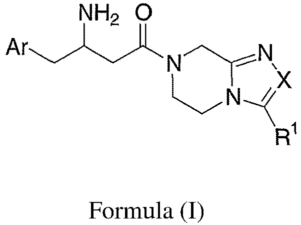 wherein:
Ar is phenyl which is unsubstituted or substituted with 1 to 5 substituents which are similar or different and independently selected from the group consisting of:
   (1) halogen,
   (2) C₁-C₆ alkyl, which is linear or branched and is unsubstituted or substituted with 1 to 5 halogens,
   (3) OC₁-C₆ alkyl, which is linear or branched and is unsubstituted or substituted with 1 to 5 halogens, and
   (4) CN;
X is selected from the group consisting of: N, and CR²;
R¹ and R² are independently selected from the group consisting of:
   (1) hydrogen,
   (2) CN,
   (3) C₁-C₁₀ alkyl, which is linear or branched and which is unsubstituted or substituted with 1 to 5 halogens or phenyl, which is unsubstituted or substituted with 1 to 5 substituents independently selected from halogen, CN, OH, R³, OR³, NHSO₂R³, SO₂R³, CO₂H, and CO₂C₁-C₆alkyl, wherein the CO₂C₁-C₆alkyl is linear or branched,
   (4) phenyl which is unsubstituted or substituted with 1 to 5 substituents independently selected from halogen, CN, OH, R³, OR³, NHSO₂R³, SO₂R³, CO₂H, and CO₂C₁-C₆alkyl, wherein the CO₂C₁-C₆ alkyl is linear or branched, and
   (5) a 5- or 6-membered heterocycle which may be saturated or unsaturated comprising 1 to 4 heteroatoms independently selected from N, S and O, the heterocycle being unsubstituted or substituted with 1 to 3 substituents independently selected from oxo, OH, halogen, C₁-C₆ alkyl, and OC₁-C₆ alkyl, wherein the C₁-C₆ alkyl and OC₁-C₆ alkyl are linear or branched and optionally substituted with 1 to 5 halogens;
R³ is C₁-C₆ alkyl, which is linear or branched and which is unsubstituted or substituted with 1 to 5 groups independently selected from halogen, CO₂H, and CO₂C₁-C₆ alkyl, wherein the CO₂C₁-C₆ alkyl is linear or branched.

The DPP-IV is known to have numerous substrates and acts by clipping dipeptides from the penultimate position of its substrates. Therefore, any peptide containing the cleavable amino acid sequence at their penultimate position is a potential substrate of DPP-IV.

For example, substrates of DPP-IV may be cytokines such as Stromal Cell-Derived Factor-1α (SDF-1 also named CXCL12 for C-X-C motif chemokine 12 ) ; growth factors, neuropeptides such as the Neuropeptide Y (NPY) and the peptide YY (PYY), the neurotransmitter substance P, the Brain Natriuretic Peptide (BNP) notably responsible for vasodilation, or Pituitary Adenylate Cyclase-Activating Polypeptide (PACAP); Regulated on Activation Normal T-Cell Expressed and Secreted (RANTES, also named CCL5) ; Eotaxin (also named CCL11) ; or incretin hormones such as glucagon-like peptide-1 (GLP-1) and glucose-dependent insulinotropic peptide or gastric inhibitory peptide (GIP).

The inhibitor of DPP-IV may alter the catalytic properties of DPP-IV and, therefore, slow down the reaction rate, or in some cases, even stop the catalysis. The inhibitor of DPP-IV may block or distort the active site of DPP-IV.

Said inhibitor of DPP-IV may be: competitive, noncompetitive, or uncompetitive.

A competitive inhibitor is defined herein as a compound structurally similar to the substrate that by its presence competes with the substrate for the active sites of the DPP-IV, thus obstructing the access of the substrate to the active site, and therefore slows down the reaction.

A noncompetitive inhibitor is defined herein as a compound that binds to the enzyme at sites distinct from the substrate binding site and alters the enzyme configuration therefore blocking or impeding the access of the substrate to the active site and/or inhibiting the catalytic action of DPP-IV.

An uncompetitive inhibitor is defined herein as a compound that binds to the enzyme-substrate complex at high concentrations of substrate, but does not bind at very low concentrations, suggesting that the binding site for the inhibitor is available only when the enzyme-substrate complex has been formed.

The inhibitor of DPP-IV may be a compound whose structure is similar to that of the substrate or product of the target enzyme and which forms either a covalent or noncovalent bond to an active site residue.

The inhibitor of DPP-IV may bond to DPP-IV in a reversible or irreversible manner.

The inhibitor of DPP-IV may inhibit the enzyme via generally competing with its substrate for binding to the active site of DPP-IV.

The inhibitor of DPP-IV may mimic the structural features of the DPP-IV's substrates, or have a chemical structure that resembles the transition state of a substrate molecule of DPP-IV.

In some embodiments, the inhibitor of DPP-IV enzyme is selected from the group comprising or consisting of alogliptin, anagliptin, besigliptin, denagliptin, evogliptin, gemigliptin, gosogliptin, linagliptin, omarigliptin, saxagliptin, teneligliptin, trelagliptin, vildagliptin, a compound of formula (I), and any pharmaceutically acceptable salts, solvates and mixtures thereof, wherein said formula (I) is as follows: 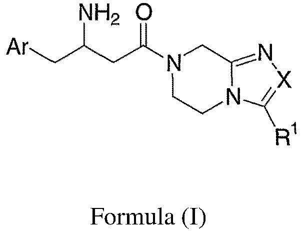 wherein:
Ar is phenyl which is unsubstituted or substituted with 1 to 5 substituents which are similar or different and independently selected from the group consisting of:
   (1) halogen,
   (2) C₁-C₆ alkyl, which is linear or branched and is unsubstituted or substituted with 1 to 5 halogens,
   (3) OC₁-C₆ alkyl, which is linear or branched and is unsubstituted or substituted with 1 to 5 halogens, and
   (4) CN;
X is selected from the group consisting of: N, and CR²;
R¹ and R² are independently selected from the group consisting of:
   (1) hydrogen,
   (2) CN,
   (3) C₁-C₁₀ alkyl, which is linear or branched and which is unsubstituted or substituted with 1 to 5 halogens or phenyl, which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched,
   (4) phenyl which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched, and
   (5) a 5- or 6-membered heterocycle which may be saturated or unsaturated comprising 1 to 4 heteroatoms independently selected from N, S and O, the heterocycle being unsubstituted or substituted with 1 to 3 substituents independently selected from oxo, OH, halogen, C₁-C₆ alkyl, and OC₁-C₆ alkyl, wherein the C₁-C₆ alkyl and OC₁-C₆ alkyl are linear or branched and optionally substituted with 1 to 5 halogens;
R³ is C₁-C₆ alkyl, which is linear or branched and which is unsubstituted or substituted with 1 to 5 groups independently selected from halogen, CO₂H, and CO₂C₁-C₆alkyl, wherein the CO₂C₁-C₆alkyl is linear or branched.

In one embodiment, the compound of formula (I) is as follows:
- Ar is phenyl which is unsubstituted or substituted with 1 to 5 substituents which are similar or different and independently selected from the group consisting of fluoro, bromo and CF3;
- R¹ is selected from the group consisting of: hydrogen and C₁-C₆ alkyl which is linear or branched and which is unsubstituted or substituted with phenyl or 1 to 5 fluoro;
- X is selected from the group consisting of: N, and CR²;
- R² is selected from the group consisting of: hydrogen, C₁-C₆ alkyl which is linear or branched and which is unsubstituted or substituted with 1 to 5 fluoro, and phenyl which is unsubstituted or substituted with 1 to 3 substituents independently selected from fluoro, OCH₃, and OCF₃.

In one embodiment, the compound of formula (I) is as follows:
- Ar is selected from the group consisting of: phenyl, 2-fluorophenyl, 3,4-difluorophenyl, 2,5-difluorophenyl, 2,4,5-trifluorophenyl, 2-fluoro-4-(trifluoromethyl)phenyl, and 4-bromo-2,5-difluorophenyl;
- R¹ is selected from the group consisting of: hydrogen, methyl, ethyl, CF₃, CH₂CF₃, CF₂CF₃, phenyl, and benzyl;
- X is selected from the group consisting of: N, and CR²;
- R² is selected from the group consisting of: hydrogen, methyl, ethyl, CF₃, CH₂CF₃, CF₂CF₃phenyl, (4-methoxy)phenyl, (4-trifluoromethoxy)phenyl, 4-fluorophenyl, and 3,4-difluorophenyl.

In one embodiment formula (I) is as follows:
- Ar is selected from the group consisting of: phenyl, 2-fluorophenyl, 3,4-difluorophenyl, 2,5-difluorophenyl, 2,4,5-trifluorophenyl, 2-fluoro-4-(trifluoromethyl)phenyl, and 4-bromo-2,5-difluorophenyl;
- R¹ is selected from the group consisting of: hydrogen and CF₃;
- X is selected from the group consisting of: N, and CR²;
- R² is selected from the group consisting of: CF₃ and CF₂CF₃.

In one embodiment, the inhibitor of DPP-IV enzyme is alogliptin. Alogliptin corresponds to the compound having the following formula

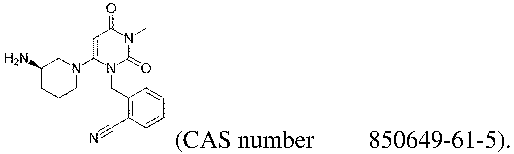

In one embodiment, the inhibitor of DPP-IV enzyme is anagliptin. Anagliptin corresponds to the compound having the following formula:

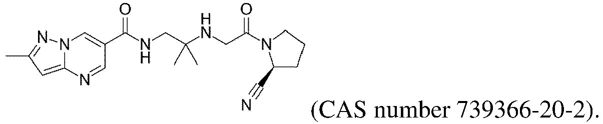

In one embodiment, the one inhibitor of DPP-IV enzyme is besigliptin. Besigliptin corresponds to the compound having the following formula:

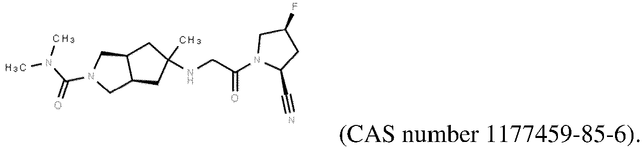

In one embodiment, the inhibitor of DPP-IV enzyme is denagliptin. Denagliptin corresponds to the compound having the following formula:

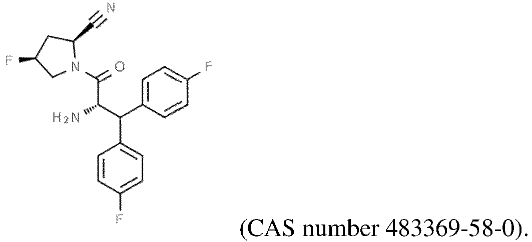

In one embodiment, the inhibitor of DPP-IV enzyme is evogliptin. Evogliptin corresponds to the compound having the following formula:

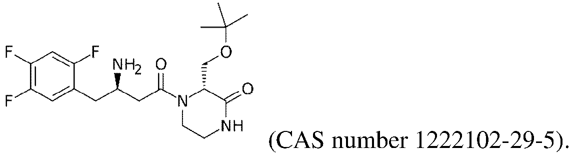

In one embodiment, the inhibitor of DPP-IV enzyme is gemigliptin. Gemigliptin corresponds to the compound having the following formula:

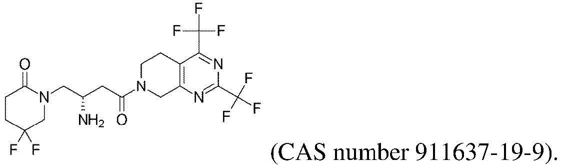

In one embodiment, the inhibitor of DPP-IV enzyme is gosogliptin. Gosogliptin corresponds to the compound having the following formula:

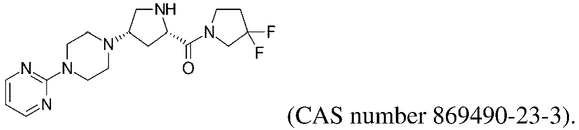

In one embodiment, the inhibitor of DPP-IV enzyme is linagliptin. Linagliptin corresponds to the compound having the following formula:

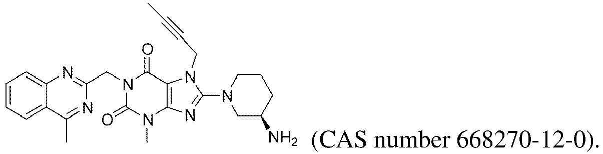

In one embodiment, the inhibitor of DPP-IV enzyme is omarigliptin. Omarigliptin corresponds to the compound having the following formula:

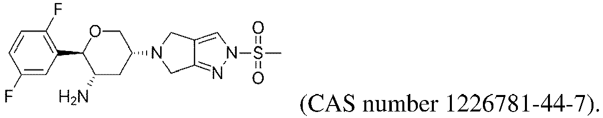

In one embodiment, the inhibitor of DPP-IV enzyme is saxagliptin. Saxagliptin corresponds to the compound having the following formula:

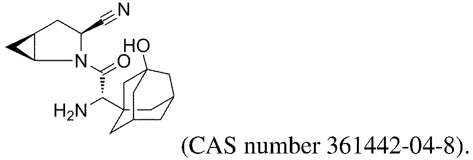

In one embodiment, the inhibitor of DPP-IV enzyme is sitagliptin. Sitagliptin corresponds to the compound having the following formula:

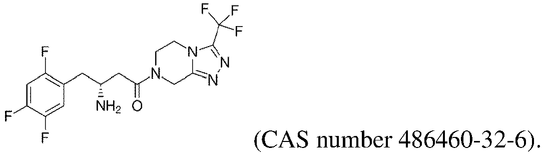

In one embodiment, the inhibitor of DPP-IV enzyme is teneligliptin. Teneligliptin corresponds to the compound having the following formula:

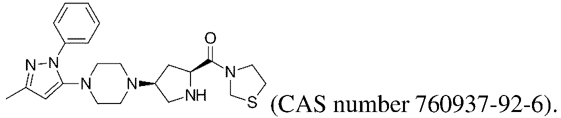

In one embodiment, the inhibitor of DPP-IV enzyme is trelagliptin. Trelagliptin corresponds to the compound having the following formula:

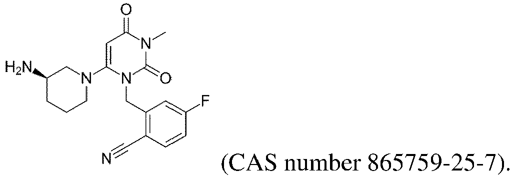

In one embodiment, the inhibitor of DPP-IV enzyme is vildagliptin. Vildagliptin corresponds to the compound having the following formula:

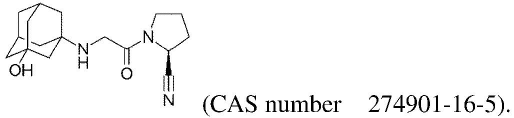

In some embodiments, the inhibitor of DPP-IV enzyme is selected from the group comprising or consisting of alogliptin, anagliptin, besigliptin, denagliptin, evogliptin, gemigliptin, gosogliptin, linagliptin, omarigliptin, saxagliptin, sitagliptin, teneligliptin, trelagliptin, vildagliptin, and any pharmaceutically acceptable salts, solvates and mixtures thereof.

In some embodiments, the inhibitor of DPP-IV enzyme is selected from the group consisting of: alogliptin, linagliptin, saxagliptin, sitagliptin, vildagliptin and any pharmaceutically acceptable salts, solvates and mixtures thereof.

In some embodiments, the inhibitor of DPP-IV enzyme is sitagliptin or any pharmaceutically acceptable salts or solvates thereof.

In some embodiments, the inhibitor of DPP-IV enzyme is to be administered in combination with an additional therapy.

In some embodiments, the additional therapy is selected from the group comprising or consisting of farnesoid X receptor (FXR) agonists, fibroblast growth factor 19 (FGF 19) mimetics, peroxisome proliferator-activated receptor (PPAR) agonists, ursodeoxycholic acid (UDCA), obeticholic acid, 24-norursodeoxycholic (nor-UDCA), microbiome-altering therapies, anti-fibrotic therapies, immunomodulatory therapies and and immunosuppressive drugs such as tacrolimus, rituximab, ustekinumab, abatacept, baricitinib, azathioprine, and mycophenolate mofetil (MMF).

In some embodiments, the additional therapy is a microbiome-altering therapy. In some embodiments, the additional therapy is an anti-fibrotic therapy. In some embodiments, the additional therapy is an immunomodulatory therapy. In some embodiments, the additional therapy is an immunosuppressive therapy; for example selected from tacrolimus, rituximab, ustekinumab, abatacept, baricitinib, azathioprine, and mycophenolate mofetil (MMF).

In some embodiments, the additional therapy is ursodeoxycholic acid, corticosteroids or other immunomodulatory therapies.

In some embodiments, the inhibitor of DPP-IV enzyme is to be administered as a monotherapy. In particular, in some embodiments, the inhibitor of DPP-IV enzyme is the sole active ingredient for use in treating or preventing an autoimmune liver disease as described herein, that is administered to the subject.

Thus, in some embodiments, the inhibitor of DPP-IV enzyme is to be administered as a monotherapy for treating an autoimmune liver disease as described herein.

In some embodiments, the autoimmune liver disease is autoimmune hepatitis (AIH). As used herein, AIH is a disease characterized by elevation of aminotransferases, presence of anti-nuclear antibody or anti-smooth muscle antibody, elevated immunoglobulin G (IgG), and interface hepatitis/plasma-lymphocytic inflammation. It may progress to liver cirrhosis and liver failure.

In some embodiments, the autoimmune liver disease is a cholestatic liver disease. In some embodiments, the autoimmune liver disease as described herein is an acute cholestatic liver disease. In some embodiments, the autoimmune liver disease as described herein is a chronic cholestatic liver disease.

As used herein, cholestatic liver diseases (CLDs) refer to a group of diseases characterized by jaundice and cholestasis as the main presentation, which can lead to end-stage liver disease, cirrhosis, and liver-related complications.

In some embodiments, the chronic cholestatic liver disease is primary biliary cholangitis (PBC) and/or primary sclerosing cholangitis (PSC).

In some embodiments, the chronic cholestatic liver disease is primary biliary cholangitis (PBC).

In some embodiments, the chronic cholestatic liver disease is primary sclerosing cholangitis (PSC).

In some embodiments, the autoimmune liver disease is autoimmune cholangitis (AIC).

In some embodiments, the autoimmune liver disease is autoimmune sclerosing cholangitis (ASC).

In some embodiments, the autoimmune liver disease is IgG4-related autoimmune cholangitis (IAC).

In some embodiments, the autoimmune liver disease as described herein is selected from the group comprising or consisting of autoimmune hepatitis, PBC, PSC, AIC, ASC, IAC and a combination thereof. It will be understood to the skilled artisan in the art that a single subject may suffer from one, two, or three of these diseases.

In some embodiments, the autoimmune liver disease is AIH and/or a cholestatic liver disease, in particular a chronic cholestatic liver, and more particularly PBC and/or PSC.

In some embodiments, the autoimmune liver disease is an overlapping syndrome comprising or consisting of AIH and PBC.

In some embodiments, the autoimmune liver disease is an overlapping syndrome comprising or consisting of AIH and PSC.

In some embodiments, the autoimmune liver disease is an overlapping syndrome comprising or consisting of AIH and PBC and PSC.

In some embodiments, the autoimmune liver disease is an overlapping syndrome comprising or consisting of PBC and PSC.

In some embodiments, the subject as described herein is a male. In some embodiments, the subject as described herein is a female.

In some embodiments, the subject as described herein is a child, *i.e.* with an age below 13 years. In some embodiments, the subject as described herein is a teenage, *i.e.* with an age between 13 years (included) and 18 years (not included). In some embodiments, the subject as described herein is an adult, *i.e.* with an age of 18 years or older.

In some embodiments, the subject as described herein is suffering from or diagnosed with an autoimmune liver disease as described herein.

In some embodiments, the subject as described herein is suffering from or diagnosed with an AIH. Means for diagnosing AIH are well known by the skilled artisan in the art and include, without limitation, blood tests or liver biopsy.

In some embodiments, the subject as described herein is suffering from or diagnosed with a cholestatic liver disease as described herein, preferably PSC and/or PBC. Means for diagnosing a cholestatic liver disease are well known by the skilled artisan in the art and include, radiologic tests by ultrasound, computerized tomography scan, magnetic resonance cholangiography, and liver biopsy.

In some embodiments, the subject in need thereof is symptomatic, i.e. has symptoms of autoimmune liver diseases as described herein.

Examples of symptoms of autoimmune hepatitis include, without limitation: abnormal fatigue, abdominal pain, jaundice, itching, spider angiomas.

Examples of symptoms of cholestatic liver diseases include, without limitation: having clay-colored or pale stools, producing dark urine, the inability to digest certain foods, itching, nausea or vomiting, pain in the right upper part of the abdomen, yellow skin or yellow eye, abnormal fatigue.

In some embodiments, the subject as described herein is asymptomatic, *i.e.* having no symptoms.

In some embodiments, the subject as described herein presents a high risk of rapid disease progression and/or a high risk of cholangiocarcinoma onset.

Indicators and symptoms of presenting a higher risk of rapid disease progression and/or a higher risk of cholangiocarcinoma onset may be: older age of diagnostic, male sex, smoking, alcohol consumption, jaundice, weight loss, portal hypertension, extended biliary involvement, ulcerative colitis, history of colorectal malignancy, history of variceal bleeding, high serum levels of alkaline phosphatase, total bilirubine, IgG4 and/or platelet counts.

In some embodiments, the subject as described herein is in the early stage of the disease (e.g. PBC or PSC). In some embodiments, the subject as described herein is in the intermediate stage of the disease (e.g. PBC or PSC). In some embodiments, the subject as described herein is in the late stage of the disease (e.g. PBC or PSC).

Early stages of PBC may be, in particular, characterized by normal levels of albumin and bilirubin, intermediate stage by abnormal level of bilirubin or albumin and late stage by abnormal level of both albumin and bilirubin.

Early, intermediate and late stages of PSC may be evaluated according to the measurement of fibrosis, bile duct loss and/or features of chronic cholestasis.

In some embodiments, the subject as described herein has been treated with an additional therapy. In some embodiments, the subject as described herein is under treatment with an additional therapy. In some embodiments, the subject as described herein will be treated, in the future, with an additional therapy.

In some embodiments, the additional therapy is as described herein.

This invention further relates to a pharmaceutical composition for use in treating or preventing an autoimmune liver disease comprising, consisting essentially of or consisting of at least one inhibitor of DPP-IV enzyme as described therein.

As used herein, the expression "consisting essentially of" means that the inhibitor of DPP-IV enzyme as described herein is the only agent with a biologic or therapeutic activity within said composition.

The pharmaceutical composition may further comprise at least one pharmaceutically acceptable excipient.

Examples of pharmaceutically acceptable excipients that may be used in the compositions of the present invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat.

In one embodiment, the pharmaceutical compositions according to the present invention comprise vehicles which are pharmaceutically acceptable for a formulation capable of being injected to a subject. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The present invention also relates to a medicament comprising, consisting essentially of or consisting at least one inhibitor of DPP-IV as described herein for use in the treatment or prevention of an autoimmune liver disease in a subject in need thereof.

The present invention further relates to the use of at least one inhibitor of DPP-IV, a composition or a pharmaceutical composition as described herein in the manufacture of a medicament for the treatment or prevention of an autoimmune liver disease in a subject in need thereof.

In some embodiments, the autoimmune liver disease is as described herein.

In some embodiments, the inhibitor of DPP-IV, the composition, the pharmaceutical composition, or the medicament as described herein is administered once, twice or three times a day.

The inhibitor of DPP-IV, the composition, the pharmaceutical composition, or the medicament as described herein may be formulated for administration to the subject in need thereof.

The inhibitor of DPP-IV, the composition, the pharmaceutical composition, or the medicament as described herein may be administered via parenteral route, preferably by injection, more preferably by intravenous, intramuscular, subcutaneous or intradermal route. The inhibitor of DPP-IV, the composition, the pharmaceutical composition, or the medicament as described herein may be administered via enteral route, such as oral route.

Examples of forms adapted for injection include, but are not limited to, solutions, such as, for example, sterile aqueous solutions, gels, dispersions, emulsions, suspensions, solid forms suitable for using to prepare solutions or suspensions upon the addition of a liquid prior to use, such as, for example, powder, liposomal forms and the like.

Sterile injectable forms of the pharmaceutical composition of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Examples of forms suitable for oral administration include, but are not limited to, tablets (including sustained-release tablets), hard capsules, powders, pills (including sugar-coated pills), capsules (including soft gelatin capsules), oral suspensions, oral solutions, and other similar forms.

All the features described above related to the inhibitor of DDP-IV enzyme for use in treating or preventing an autoimmune liver disease according to the invention apply *mutatis mutandis* for the composition, pharmaceutical composition or medicament as described herein.

This invention also relates to a method for treating or preventing (e.g. reducing the likelihood of occurrence or reoccurrence of) an autoimmune liver disease as described herein by administering to a subject in need thereof a therapeutically effective amount of at least one inhibitor of DPP-IV enzyme.

The method as described herein may further comprise a step of administering another therapy to the subject. In particular, the other therapy may be as described herein.

All the features described above related to the inhibitor of DDP-IV enzyme for use in treating or preventing an autoimmune liver disease according to the invention apply *mutatis mutandis* for the method(s) described herein.

### BRIEF DESCRIPTION OF THE DRAWINGS

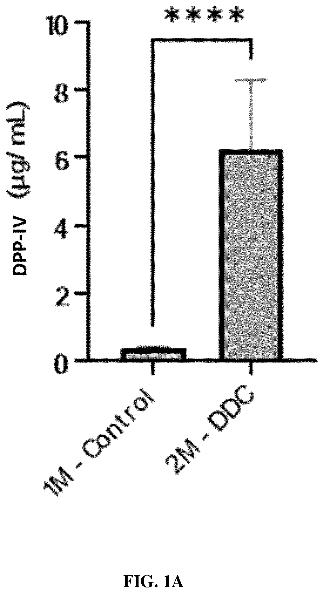

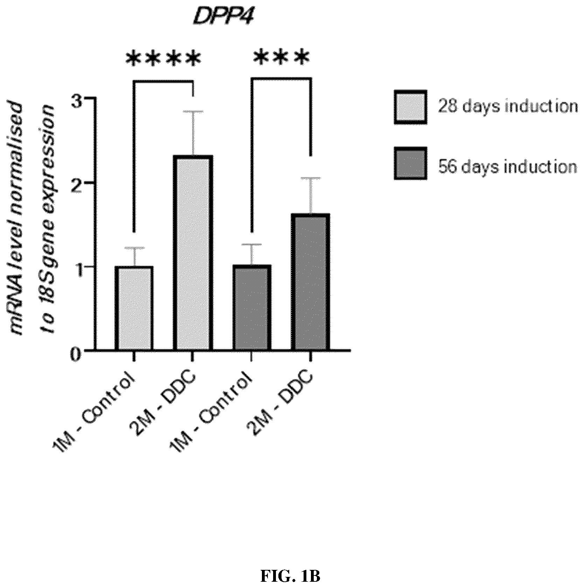

**Figure 1** is a combination of histograms showing that DPP-IV is expressed in mice with DDC diet. **Figure 1A** is a histogram showing the concentration of DPP-IV in the serum of mice from a control group (1M - control) and from a group fed with DDC diet (2M-DDC). **Figure 1B** is a histogram showing the mRNA levels of DPP-IV normalized to 18S mRNA levels in the livers of mice from a control group (1M - control) and from a group fed with DDC diet (2M-DDC), after 28 days of induction with DDC diet and after 56 days of induction with DDC diet.

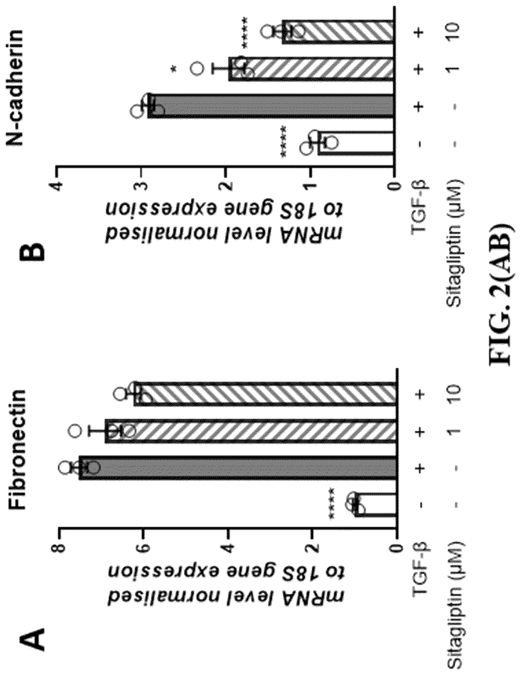

**Figure 2** is a group of histograms showing the effect of sitagliptin on mesenchymal marker genes. **Figure 2** presents the mRNA levels of fibronectin (Fig. 2A) and N-cadherin (Fig. 2B) normalized to 18S mRNA levels in TFK-1 cells, in the following conditions: control without TGF-β; control with induction by TGF-β; treatment with sitagliptin 1µM and treatment with sitagliptin 10µM (the significative differences are indicated in the graph with * = p <0.05; ** = p <0.01; *** = p < 0.001; **** = p <0.0001).

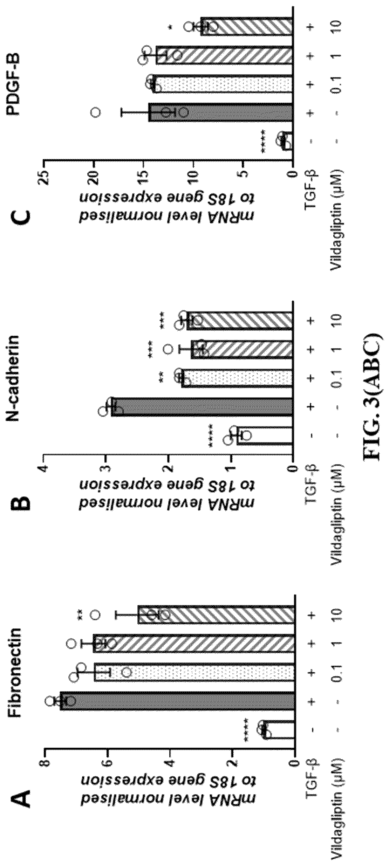

**Figure 3** is a group of histograms showing the effect of vildagliptin on mesenchymal marker genes. **Figure 3** presents the mRNA levels of fibronectin (Fig. 3A), N-cadherin (Fig. 3B) and PDGF-B (Fig. 3C) normalized to 18S mRNA levels in TFK-1 cells, in the following conditions: control without TGF-β; control with induction by TGF-β; treatment with vildagliptin 0.1µM; treatment with vildagliptin 1µM and treatment with vildagliptin 10µM (the significative differences are indicated in the graph with * = p <0.05; ** = p <0.01; *** = p < 0.001; **** = p <0.0001).

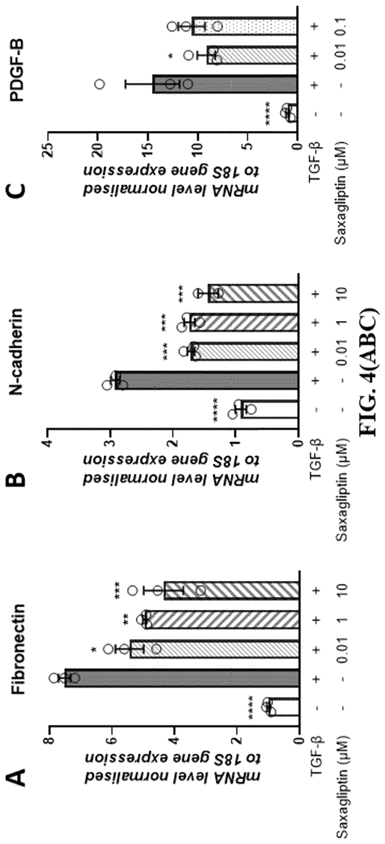

**Figure 4** is a group of histograms showing the effect of saxagliptin on mesenchymal marker genes. **Figure 4** presents the mRNA levels of fibronectin (Fig. 4A), N-cadherin (Fig. 4B) and PDGF-B (Fig. 4C) normalized to 18S mRNA levels in TFK-1 cells, in the following conditions: control without TGF-β; control with induction by TGF-β; treatment with saxagliptin 0.01µM; treatment with saxagliptin 0.1µM; treatment with saxagliptin 1µM and treatment with saxagliptin 10µM (the significative differences are indicated in the graph with * = p <0.05; ** = p <0.01; *** = p < 0.001; **** = p <0.0001).

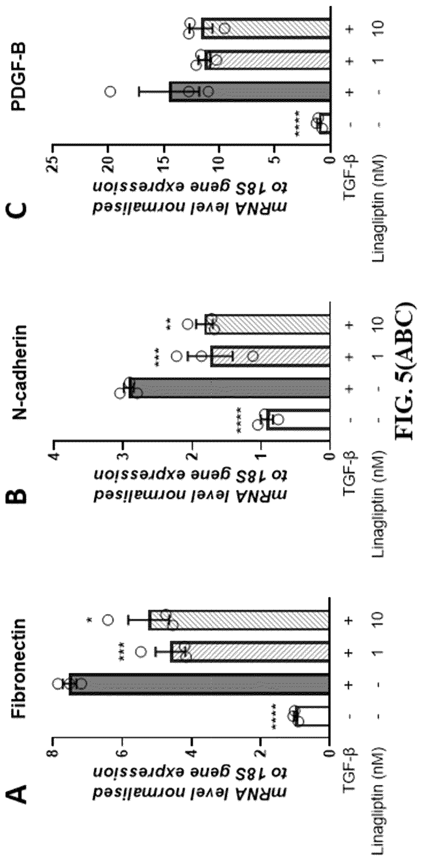

**Figure 5** is a group of histograms showing the effect of linagliptin on mesenchymal marker genes. **Figure 5** presents the mRNA levels of fibronectin (Fig. 5A), N-cadherin (Fig. 5B) and PDGF-B (Fig. 5C) normalized to 18S mRNA levels in TFK-1 cells, in the following conditions: control without TGF-β; control with induction by TGF-β; treatment with linagliptin 1nM; treatment with linagliptin 10nM; (the significative differences are indicated in the graph with * = p <0.05; ** = p <0.01; *** = p < 0.001; **** = p <0.0001).

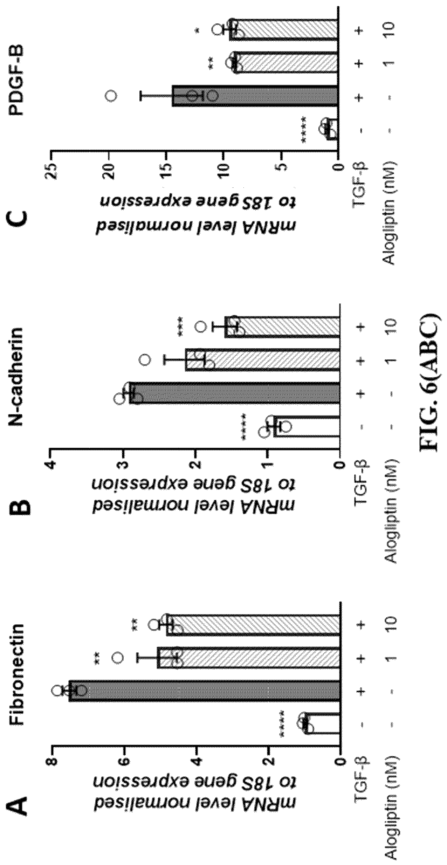

**Figure 6** is a group of histograms showing the effect of alogliptin on mesenchymal marker genes. Figure 6 presents the mRNA levels of fibronectin (Fig. 6A), N-cadherin (Fig. 6B) and PDGF-B (Fig. 6C) normalized to 18S mRNA levels in TFK-1 cells, in the following conditions: control without TGF-β; control with induction by TGF-β; treatment with alogliptin 1nM; treatment with alogliptin 10nM; (the significative differences are indicated in the graph with * = p <0.05; ** = p <0.01; *** = p < 0.001; **** = p <0.0001).

### EXAMPLES

The present invention is further illustrated by the following examples.

### Example 1: DPP-IV is significantly increased in vivo in a PSC model

Two groups of 7-week old SWISS male mice were allocated for this study: a control group 1M (n=12) and a DDC group 2M (n=24). The control group was fed standard diet while a PSC model was induced in the DDC group by feeding the animals a standard diet supplemented with 0.1% 3,5-diethoxycarbonyl-1,4 dihydrocollidine (DDC diet). During the study, mortality and morbidity observations were performed, and the clinical score and body weight were measured. The guidelines used were to euthanize mice reaching a body weight loss superior to 20% of their initial body weight during 24h and/or reaching humane endpoints, but none met these criteria.

On day 28, half of the mice from each group was euthanized, and their livers were sampled for measuring the mRNA levels of DPP-IV in the livers (Example 1B).

On day 56, the other half of the mice from each group was sampled for blood for measuring the DPP-IV expression in serum (Example 1A) and euthanized. Their livers were also sampled for measuring the mRNA levels of DPP-IV in the livers (Example 1B).

### Example 1A. - In the serum

### Materials and Methods

Two groups of 7-week-old SWISS male mice were allocated for this study: a control group 1M (n=6) and a DDC group 2M (n=12). The control group was fed standard diet while a PSC model was induced in the DDC group by feeding the animals a standard diet supplemented with 0.1% 3,5-diethoxycarbonyl-1,4 dihydrocollidine (DDC diet).

During the study, mortality and morbidity observations, clinical score and body weight measurements were performed; guidelines were to euthanize mice reaching a body weight loss superior to 20% of their initial body weight during 24h and/or reaching humane endpoints, but none met these criteria. On day 56, all mice were sampled for serum and euthanized. Concentrations of serum DPP-IV were quantified.

### Results

As shown in Figure 1, serum DDP-IV level was only detected at low concentrations in Control mice (0.362 ± 0.054 µg/mL) while those fed with DDC diet showed a significant increase in DPP4 level (6.208 ± 2.014 µg/mL; <0.0001).

In conclusion, blood circulating DPP-IV level is significantly increased in a chemically-induced cholangitis model of PSC.

### Example 1B. - In the liver

### Materials and Methods

On day 28, 6 mice from control group 1M and 12 mice from DDC group 2M were euthanized, and their livers were sampled, snap-frozen, and kept at -80°C until analyses. Similarly, on day 56, 6 mice from control group 1M and 12 mice from DDC group 2M were euthanized, and their livers were sampled, snap-frozen, and kept at -80°C until analyses.

Total RNA was extracted from the snap-frozen livers, quantified and reverse-transcribed into complementary DNA (cDNA), and DPP-IV gene expression analysis was performed using quantitative polymerase chain reaction (qPCR) with SYBR Green PCR Master Mix. mRNA levels were normalized to the expression of the housekeeping gene 18S (Figure 1B).

### Results

A significant increase in DPP-IV mRNA level was observed in the DDC-feeding group compared to control animals, both after 28 days of the DDC diet (2.32-fold increase, p < 0.0001 ) and after 56 days of the DDC diet (1.63-fold increase, p < 0.001).

In conclusion, DPP-IV gene expression in the liver is significantly increased in a chemically-induced cholangitis model of PSC.

### Example 2: Effect of DPP-IV inhibitors on an in vivo mouse model of cholestatic diseases 3,5-Diethoxycarbonyl-1,4-Dihydrocollidine Diet (DDC diet)

### Materials and Methods

### DDC diet model

Six-week-old SWISS male mice are procured from Janvier Labs (France), and all diets are provided by Ssniff (Germany). The mice are divided into distinct groups (n=10 mice/group) to ensure weight homogeneity among groups.

The experimental design includes the following groups:
- Control group, fed a standard diet for 8 weeks;
- DDC group, fed with DDC diet (standard diet supplemented with 0.1% 3,5-diethoxycarbonyl-1,4 dihydrocollidine) for 8 weeks;
- Treatment groups, initially fed DDC diet for 4 weeks, followed by feeding of DDC diet and intragastric delivery of a DPP-IV inhibitor suspended in vehicle (at different concentrations) up to five times a week for an additional 4 weeks.

Mice in the Control and DDC groups also receive intragastric vehicle administration up to five times a week during the latter 4-week period. Mice are euthanized 8 weeks after the beginning of the experiment, and blood and tissue samples were collected for analysis.

Alternatively, animals first receive treatment (or vehicle; up to five times a week) as soon as they are fed DDC (or control) diet. Mice are then euthanized 4 or 8 weeks after the beginning of the experiment, and blood and tissue samples are collected for analysis.

### Determination of serologic and tissue markers indicative of liver disease and fibrosis development

Part of the serum isolated from fresh blood is stored at -80°C and assayed within 3 months for Aspartate Transaminase, Alkalin Phosphatase and total Bilirubin using an automated chemilumiscence assay.

Another part of the collected serum is stored at -80°C and assayed within 3 months for total levels of Hyaluronic acid, Tissue MetalloProteinase Inhibitor-1 and DPP4, using ELISA kits; and for total biliary acids level using an enzymatic colorimetric assay.

Left liver lobes are fixed in 4% paraformaldehyde and embedded in paraffin for hematoxylin-eosin staining and, Sirius red staining, followed by fibrosis scoring. The caudate and right lobes are snap-frozen in liquid nitrogen, stored at -80°C and assayed within 3 months (1) for COL1A2, FN1, VIM, SNAIl, CDH2, ACTA2, SPP1, TGFB1, TNFA, TIMP1 expression, through qRT-PCR, using SYBR Green PCR Master Mix; (2) for total levels of α-SMA, TGF-β, TNF-α and γGT using ELISA kits; (3) for total biliary acids level using an enzymatic colorimetric assay.

### Example 3: DPP-IV inhibition significantly decreases epithelial-to-mesenchymal transition (EMT) in vitro in response to TGF-β treatment

### Materials and Methods

TFK-1 cells, a human cholangiocarcinoma cell line, were seeded at a density of 4.1 x 10⁴ cells per well in 24-well plates. Cells were initially cultured in RPMI 1640 medium supplemented with 10% fetal bovine serum (FBS) for 24 hours. Subsequently, the medium was replaced with RPMI 1640 containing 1% FBS, in which cells were maintained for the remainder of the experiment. At 48 hours post-seeding, cells were treated with 10 ng/mL of transforming growth factor-beta (TGF-β) for 24 hours, either alone or in combination with varying concentrations of DPP-IV inhibitors (sitagliptin, vildagliptin, saxagliptin, linagliptin or alogliptin). After the treatment period, cells were harvested, and total RNA was extracted and stored at -80°C for later analysis.

Within one week of extraction, the RNA was quantified, reverse-transcribed into complementary DNA (cDNA), and gene expression analysis was performed using quantitative polymerase chain reaction (qPCR) with SYBR Green PCR Master Mix. The target genes included those encoding fibronectin (FN1), N-cadherin (CH2), and platelet-derived growth factor B (PDGFB). Gene expression levels were normalized to the expression of the housekeeping gene 18S using the *ddCt* method. For each gene, all treatment conditions were compared to treatment with TGF-β alone using multiple-comparison one-way ANOVA test.

### Results

As shown in Figures 2 to 6, exposure to TGF-β for 24 hours resulted in a significant upregulation of mesenchymal marker genes in TFK-1 cells: a 7.53-fold increase in fibronectin expression compared to fibronectin expression without TGF- β for 24 hours (Fig.2A, 3A, 4A, 5A and 6A), a 2.92-fold increase in N-cadherin expression compared to N-cadherin expression without TGF- β for 24 hours (2B, 3B, 4B, 5B and 6B), and a 14.53-fold increase in PDGF-B expression compared to PDGF-B expression without TGF- β for 24 hours (Fig. 3C, 4C, 5C and 6C).

Co-treatment with sitagliptin (Fig.2), vildagliptin (Fig.3), saxagliptin (Fig.4), linagliptin (Fig.5) or alogliptin (Fig.6) significantly reduced the TGF-β-induced overexpression of at least one of the three mesenchymal marker genes.

10µM of sitagliptin demonstrated selective inhibition, reducing significantly N-cadherin overexpression by 2.03-fold (Fig. 2B). Sitagliptin also showed a tendency of a decreased expression of fibronectin (Fig. 2A).

Co-treatment with vildagliptin, saxagliptin or alogliptin significantly reduced the TGF-β-induced overexpression of all three mesenchymal marker genes. Reductions in fibronectin expression were 1.49-fold, 1.38-fold, and 1.55-fold, respectively, with 10µM vildagliptin, 10nM saxagliptin and 10nM alogliptin (Fig. 3A, 4A and 6A). Similarly, N-cadherin expression was reduced by 1.71-fold, 1.70-fold, and 1.84-fold with 10µM vildagliptin, 10nM saxagliptin and 10nM alogliptin (Fig. 3B, 4B and 6B), and PDGF-B expression was reduced by 1.57-fold, 1.58-fold, and 1.53-fold, respectively with 10µM vildagliptin, 10nM saxagliptin and 10nM alogliptin (Fig. 3C, 4C and 6C).

1nM of linagliptin significantly attenuated the TGF-β-induced expression of fibronectin (1.63-fold reduction) (Fig. 5A) andN-cadherin (1.68-fold reduction) (Fig.5B). Linagliptin also showed a tendency of a decreased expression of PDGF-B (Fig. 5C).

In conclusion, DPP-IV inhibitors, particularly vildagliptin, saxagliptin and alogliptin, significantly inhibited TGF-β-induced epithelial-to-mesenchymal transition in TFK-1 cells, as evidenced by the reduction in the expression of key mesenchymal markers.

## Claims

1. An inhibitor of dipeptidyl peptidase-IV enzyme for use in treating or preventing an autoimmune liver disease (AILD), preferably a chronic cholestatic liver disease such as primary biliary cholangitis (PBC) and/or primary sclerosing cholangitis (PSC) in a subject in need thereof; wherein the inhibitor of dipeptidyl peptidase-IV enzyme is a compound selected from the group consisting of formula (I), alogliptin, anagliptin, besigliptin, denagliptin, evogliptin, gemigliptin, gosogliptin, linagliptin, omarigliptin, saxagliptin, teneligliptin, trelagliptin, vildagliptin, and any pharmaceutically acceptable salts, solvates and mixtures thereof, wherein said formula (I) is as follows: 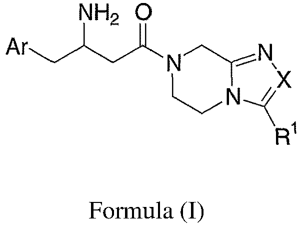 wherein:
Ar is phenyl which is unsubstituted or substituted with 1 to 5 substituents which are similar or different and independently selected from the group consisting of:
(1) halogen,
(2) C₁-C₆ alkyl, which is linear or branched and is unsubstituted or substituted with 1 to 5 halogens,
(3) OC₁-C₆ alkyl, which is linear or branched and is unsubstituted or substituted with 1 to 5 halogens, and
(4) CN;
X is selected from the group consisting of: N, and CR²;
R¹ and R² are independently selected from the group consisting of:
(1) hydrogen,
(2) CN,
(3) C₁-C₁₀ alkyl, which is linear or branched and which is unsubstituted or substituted with 1 to 5 halogens or phenyl, which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched,
(4) phenyl which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched, and
(5) a 5- or 6-membered heterocycle which may be saturated or unsaturated comprising 1 to 4 heteroatoms independently selected from N, S and O, the heterocycle being unsubstituted or substituted with 1 to 3 substituents independently selected from oxo, OH, halogen, C₁-C₆ alkyl, and OC₁-C₆ alkyl, wherein the C₁-C₆ alkyl and OC₁-C₆ alkyl are linear or branched and optionally substituted with 1 to 5 halogens;
R³ is C₁-C₆ alkyl, which is linear or branched and which is unsubstituted or substituted with 1 to 5 groups independently selected from halogen, CO₂H, and CO₂C₁-C₆ alkyl, wherein the CO₂C₁-C₆ alkyl is linear or branched.

2. The inhibitor of dipeptidyl peptidase-IV enzyme for use according to claim 1, wherein the inhibitor of dipeptidyl peptidase-IV enzyme is a compound of formula (I) or any pharmaceutically acceptable salts, solvates or mixtures thereof, wherein said formula (I) is as follows: 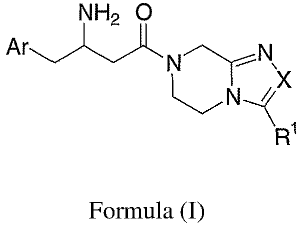 wherein:
Ar is phenyl which is unsubstituted or substituted with 1 to 5 substituents which are similar or different and independently selected from the group consisting of:
(1) halogen,
(2) C₁-C₆ alkyl, which is linear or branched and is unsubstituted or substituted with 1 to 5 halogens,
(3) OC₁-C₆ alkyl, which is linear or branched and is unsubstituted or substituted with 1 to 5 halogens, and
(4) CN;
X is selected from the group consisting of: N, and CR²;
R¹ and R² are independently selected from the group consisting of:
(1) hydrogen,
(2) CN,
(3) C₁-C₁₀ alkyl, which is linear or branched and which is unsubstituted or substituted with 1 to 5 halogens or phenyl, which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched,
(4) phenyl which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched, and
(5) a 5- or 6-membered heterocycle which may be saturated or unsaturated comprising 1 to 4 heteroatoms independently selected from N, S and O, the heterocycle being unsubstituted or substituted with 1 to 3 substituents independently selected from oxo, OH, halogen, C₁-C₆alkyl, and OC₁-C₆alkyl, wherein the C₁-C₆ alkyl and OC₁-C₆ alkyl are linear or branched and optionally substituted with 1 to 5 halogens;
R³ is C₁-C₆ alkyl, which is linear or branched and which is unsubstituted or substituted with 1 to 5 groups independently selected from halogen, CO₂H, and CO₂C₁-C₆ alkyl, wherein the CO₂C₁-C₆ alkyl is linear or branched.

3. The inhibitor of dipeptidyl peptidase-IV enzyme for use according to claim 1 or claim 2, wherein in formula (I):
- Ar is phenyl which is unsubstituted or substituted with 1 to 5 substituents which are similar or different and independently selected from the group consisting of fluoro, bromo and CF₃;
- R¹ is selected from the group consisting of: hydrogen, C₁-C₆ alkyl which is linear or branched and which is unsubstituted or substituted with phenyl or 1 to 5 fluoro;
- X is selected from the group consisting of: N, and CR²;
- R² is selected from the group consisting of: hydrogen, C₁-C₆ alkyl which is linear or branched and which is unsubstituted or substituted with 1 to 5 fluoro, and phenyl which is unsubstituted or substituted with 1 to 3 substituents independently selected from fluoro, OCH₃, and OCF₃.

4. The inhibitor of dipeptidyl peptidase-IV enzyme for use according to any one of claims 1 to 3, wherein in formula (I):
- Ar is selected from the group consisting of: phenyl, 2-fluorophenyl, 3,4-difluorophenyl, 2,5-difluorophenyl, 2,4,5-trifluorophenyl, 2-fluoro-4-(trifluoromethyl)phenyl, and 4-bromo-2,5-difluorophenyl;
- R¹ is selected from the group consisting of: hydrogen, methyl, ethyl, CF₃, CH₂CF₃, CF₂CF₃, phenyl, and benzyl;
- X is selected from the group consisting of: N, and CR²;
- R² is selected from the group consisting of: hydrogen, methyl, ethyl, CF₃, CH₂CF₃, CF₂CF₃phenyl, (4-methoxy)phenyl, (4-trifluoromethoxy)phenyl, 4-fluorophenyl, and 3,4-difluorophenyl.

5. The inhibitor of dipeptidyl peptidase-IV enzyme for use according to claim 3, wherein in formula (I):
- Ar is selected from the group consisting of: phenyl, 2-fluorophenyl, 3,4-difluorophenyl, 2,5-difluorophenyl, 2,4,5-trifluorophenyl, 2-fluoro-4-(trifluoromethyl)phenyl, and 4-bromo-2,5-difluorophenyl;
- R¹ is selected from the group consisting of: hydrogen and CF₃;
- X is selected from the group consisting of: N, and CR²;
- R² is selected from the group consisting of: CF₃ and CF₂CF₃.

6. The inhibitor of dipeptidyl peptidase-IV enzyme for use according to claim **2,** wherein said inhibitor of dipeptidyl peptidase-IV enzyme is selected from the group consisting of: alogliptin, anagliptin, besigliptin, denagliptin, evogliptin, gemigliptin, gosogliptin, linagliptin, omarigliptin, saxagliptin, sitagliptin, teneligliptin, trelagliptin, vildagliptin, and any pharmaceutically acceptable salts, solvates and mixtures thereof.

7. The inhibitor of dipeptidyl peptidase-IV enzyme for use according to claim **2,** wherein said inhibitor of dipeptidyl peptidase-IV enzyme is selected from the group consisting of: alogliptin, linagliptin, saxagliptin, sitagliptin, vildagliptin and any pharmaceutically acceptable salts, solvates and mixtures thereof.

8. The inhibitor of dipeptidyl peptidase-IV enzyme for use according to claim **1,** wherein said inhibitor of dipeptidyl peptidase-IV enzyme is sitagliptin or any pharmaceutically acceptable salts or solvates thereof.

9. The inhibitor of dipeptidyl peptidase-IV enzyme for use according to any one of claims **1** to **8,** wherein the inhibitor of dipeptidyl peptidase-IV enzyme is to be administered before, concomitantly with or after an additional therapeutic agent.

10. The inhibitor of dipeptidyl peptidase-IV enzyme for use according to any one of claims **1** to **9,** wherein the subject is a child, a teenager, or an adult.

11. A pharmaceutical composition for use in treating or preventing an autoimmune liver disease in a subject in need thereof; wherein the pharmaceutical composition comprises at least one inhibitor of dipeptidyl peptidase-IV enzyme, wherein said inhibitor of dipeptidyl peptidase-IV enzyme is selected from the group consisting of: alogliptin, anagliptin, besigliptin, denagliptin, evogliptin, gemigliptin, gosogliptin, linagliptin, omarigliptin, saxagliptin, teneligliptin, trelagliptin, vildagliptin, a compound of formula (I), and any pharmaceutically acceptable salts, solvates and mixtures thereof, wherein said formula (I) is as follows: 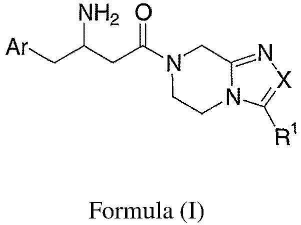 wherein:
Ar is phenyl which is unsubstituted or substituted with 1 to 5 substituents which are similar or different and independently selected from the group consisting of:
(1) halogen,
(2) C₁-C₆ alkyl, which is linear or branched and is unsubstituted or substituted with 1 to 5 halogens,
(3) OC₁-C₆ alkyl, which is linear or branched and is unsubstituted or substituted with 1 to 5 halogens, and
(4) CN;
X is selected from the group consisting of: N, and CR²;
R¹ and R² are independently selected from the group consisting of:
(1) hydrogen,
(2) CN,
(3) C₁-C₁₀ alkyl, which is linear or branched and which is unsubstituted or substituted with 1 to 5 halogens or phenyl, which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched,
(4) phenyl which is unsubstituted or substituted with 1 to 5 substituents independently selected from halogen, CN, OH, R³, OR³, NHSO₂R³, SO₂R³, CO₂H, and CO₂C₁-C₆ alkyl, wherein the CO₂C₁-C₆ alkyl is linear or branched, and
(5) a 5- or 6-membered heterocycle which may be saturated or unsaturated comprising 1 to 4 heteroatoms independently selected from N, S and O, the heterocycle being unsubstituted or substituted with 1 to 3 substituents independently selected from oxo, OH, halogen, C₁-C₆ alkyl, and OC₁-C₆ alkyl, wherein the C₁-C₆ alkyl and OC₁-C₆ alkyl are linear or branched and optionally substituted with 1 to 5 halogens;
R³ is C₁-C₆ alkyl, which is linear or branched and which is unsubstituted or substituted with 1 to 5 groups independently selected from halogen, CO₂H, and CO₂C₁-C₆ alkyl, wherein the CO₂C₁-C₆ alkyl is linear or branched.

12. A pharmaceutical composition for use according to claim **11,** further comprising at least one pharmaceutically acceptable excipient selected from the group consisting of: ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, polyacrylates, waxes, polyethylene-polyoxypropylene-block polymers, polyethylene glycol and wool fat.

13. The inhibitor of dipeptidyl peptidase-IV enzyme or the pharmaceutical composition for use according to any one of claims **1** to **12,** wherein the autoimmune liver disease is a chronic cholestatic liver disease and/or autoimmune hepatitis.

14. The inhibitor of dipeptidyl peptidase-IV enzyme or the pharmaceutical composition for use according to claim **13,** wherein the chronic cholestatic liver disease is primary biliary cholangitis (PBC) and/or primary sclerosing cholangitis (PSC).
